Europäisches Patentamt

European Patent Office

Office européen des brevets

(11) Publication number: **0 373 717**
**A1**

## EUROPEAN PATENT APPLICATION

(21) Application number: 89203145.1

(22) Date of filing: 08.12.88

(51) Int. Cl.⁵: **A61B 6/14**

(43) Date of publication of application:
**20.06.90 Bulletin 90/25**

(60) Publication number of the earlier application in
accordance with Art.76 EPC: **0 372 122**

(84) Designated Contracting States:
**CH DE ES FR GB IT LI**

(71) Applicant: **N.V. Philips' Gloeilampenfabrieken**
**Groenewoudseweg 1**
**NL-5621 BA Eindhoven(NL)**

(72) Inventor: **Molteni, Roberto**
**c/o INT. OCTROOIBUREAU B.V. Prof.**
**Holstlaan 6**
**NL-5656 AA Eindhoven(NL)**
Inventor: **Zanmarchi, Giulio**
**c/o INT. OCTROOIBUREAU B.V. Prof.**
**Holstlaan 6**
**NL-5656 AA Eindhoven(NL)**

(74) Representative: **Scheele, Edial François et al**
**INTERNATIONAAL OCTROOIBUREAU B.V.**
**Prof. Holstlaan 6**
**NL-5656 AA Eindhoven(NL)**

(54) Dental x-ray image detection system.

(57) In a dental X-ray apparatus use is made of a
fibre optics device (13) provided with an obliguely
cut end surface (12) to transmit an optical image
projected into the fibre optics device on that surface
onto a second end face (15) thereof forming on the
second end face an image with a reduced surface
area. Using a crossed cascade of two fibre optics
devices (13, 23), an image area can be reduced in
two directions.

FIG. 2

FIG.3

## Dental X-ray image detection system.

The invention relates to a dental X-ray image detection system provided with an optical sensitive sensor being fibre optically coupled to a luminescent screen.

Such a detection system is known from US 4,593,400. In this system a luminescent screen is optically coupled to a CCD matrix as optical sensor with the aid of a tapered fibre optical device such that the surface area of an X-ray image projected on the luminescent layer is reduced to a smaller surface area to be projected on the CCD matrix.

For a practical device the reduction in the dimension of the X-ray image and the sensor image should be relatively high in order to have an X-ray image large enough to encompass at least a full tooth and to have the opportunity to make use of a relatively low cost CCD device. The device disclosed in US 4,593,400 is not suited to realise such a high image reduction at acceptable costs without the risk that the image will become deteriorated in the fibre optics device due to irregularities therein as a consequence of the too strong tapering or that the fibre optics device becomes unacceptable long for practical use. A further disadvantage of the known apparatus is that the CCD matrix must be protected against direct X-ray radiation from the source as well as against X-rays scattered in the object to be examined or in parts of the apparatus. In the known apparatus the detecting device including the CCD matrix is located in direct view of the X-ray source X-ray protection has to be added between the luminescent layer and the sensor. Such a protection can easily have an adversely effect on t4e image forming.

In the known apparatus the screening is executed by incorporating X-ray absorbing material in the optical transmitting for preventing passage of X-rays surpassed the luminescent layer. Such material has a negative effect on the image transmission. Especially due to the direct view orientation also the core glass must be X-ray absorbing with all its negative effects onto the light transmission.

From EP 149 502 a fibre optically coupled X-ray detection system is known but there the need to have a small dimension in the direction of the imaging X-ray beam does not exist. The same can be said of the scanning apparatus disclosed in EP 279 293.

It is a main object mentioned disadvantages and thereto a detecting system for a dental X-ray apparatus as defined above according to the invention is charactezed in that the detection system comprises a fibre optical device having a first surface obliquely cut with respect to the fibres, introducing a surface area reduction between this surface and a second surface of the device optically connected thereto.

In a further embodiment the fibre optics device reduces the long axis of a rectangular X-ray image with about a factor 3 adapting the optical image area to a sensor surface area having a long axis substantial equal to the short axis of the X-ray image and a small axis substantial equal to a third of the long X-ray image area axis. Using two fibre optics elements in cascade each introducing a linear reduction of roughly a factor 3 a reduction of an X-ray image of about 18x24 mm$^2$ can be reduced to a sensor area of 6x8 mm$^2$ which is near to normal available relatively cheap obtainable in the shape of a CCD matrix sensor.

In a further embodiment a luminescent layer, preferably of CsI (T1) or Gd202S (Tb), is directly coupled to a first obliguely cut surface of the fibre optics device and an optical sensitive sensor preferably a CCD matrix sensor is coupled to a second surface of the fibre optical device optically coupled to the first surface.

In a further embodiment the fibre optics device is cut along two optically coupled surfaces such that substantially a 90° angle is realised between said two surfaces.

"A non-90° cutting angle for the read out surface gives an additional parameter to adapt the geometry of the read out surface to the measuring of a CCD image sensor. A cutting angle of at least 90° with the entrance surface results in an improved protection of the CCD sensor against X-rays in that no part of the sensor becomes into a straight-on radiation field of the X-ray beam. Alternatively for a 90° cutting angle the fibre optics device can be registered in the X-ray system such that also no straight-on radiation can see the sensor. A cutting angle less than 90° can result in a more compact device but than the fibre optics device must be relatively high X-ray absorbent.

In a further embodiment the cladding glass and/or the extra mural absorbent elements of the fibre optics device contain X-ray absorbing material or are made of lead glass for preventing the sensor against scattered X-rays.

Some embodiments according to the invention will be described more detailed hereinafter with reference to the drawing in which

Figure 1 shows schematically a dental X-ray apparatus provided with a detection system according to the invention, and

Figures 2 and 3 shows examples of fibre optical devices applicable therefore.

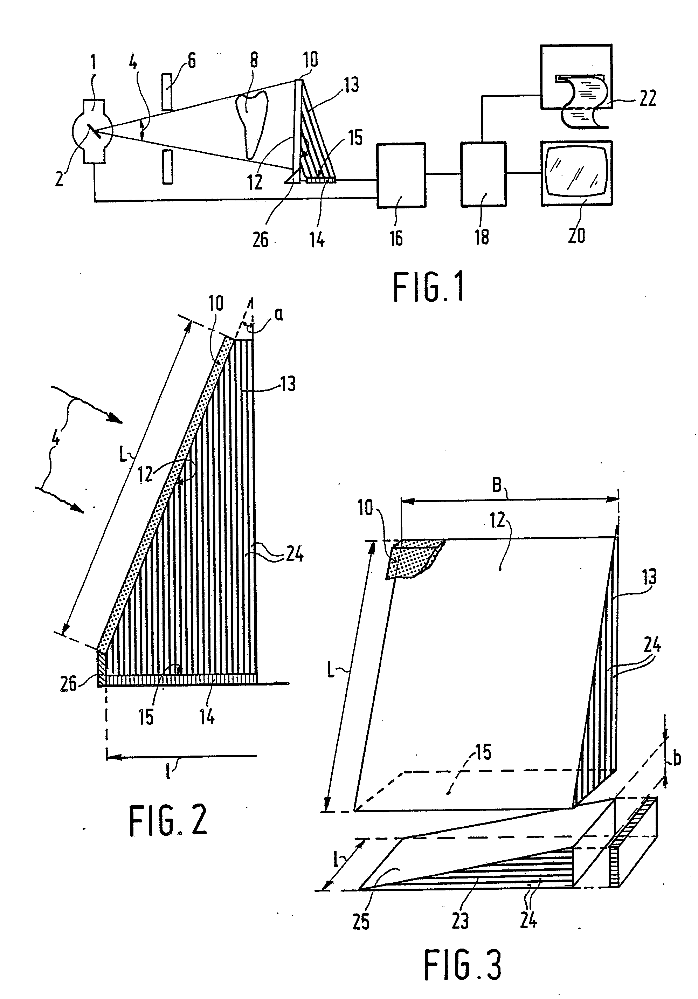

A dental X-ray apparatus as shown in Figure 1 comprises an X-ray source 1 with an anode 2 to generate an X-ray beam 4, which is limited with a diaphragm 6 to irradiate an object 8, here a tooth, to be examined. A shadow image of the object is projected on a luminescent layer 10 converting the X-ray quanta into light quanta. The luminescent layer 10 comprising for example CsI (T1 activated or Gd202s (Tb activated) is directly deposited on a fibre optics surface 12 by vapour deposition, spraying or any other applicable deposition method. Part of the luminescent light generated in the luminescent layer is projected into the fibres of a fibre optics device 13 such that a light optical image, corresponding with the X-ray image projected on the luminescent layer is transmitted by the fibre optics element to an optical sensitive sensor 14 provided in front of a second surface 15 of the fibre optical device. Preferably the sensor 14 is coupled as directly as possible to the fibre optics device in order to avoid loss of imaging light. The optical sensitive sensor preferably is shaped as an electronic matrix sensor in order to enable digital electronics reading and may be constructed as a CCD matrix sensor available on the market. Known CCD matrix sensor have, for example, a detecting surface area of 4.5 times 6.0 mm containing a matrix of about 600 times about 500 elements each measuring about 10 times 10 $\mu$m or a detecting area of aobut 13 times 9 mm composed of about 575 times 380 elements each measuring about 25 times 25 $\mu$m. The optical sensor 14 is electrically coupled to a signal reading - and signal control interface device 16 to which an image processor 18 is coupled. The image processor, in addition to the normal image processing known to be performed in this kind of apparatus such as contrast alteration, edge enhancement, pattern recognition etc., could also provide the spatial tretching in one direction on which may be necessary to reconstruct the actual geometrical proportions of the image. To the image processor a monitor 20 for displaying the image of the object or an image derived after image processing and a hard copy unit to produce if asked for a copy on paper or transparent film or a magnetically or optically registered hard copy of an image.

Figure 2 shows a fibre optics device 13 more detailed together with a luminescent layer 10 which is for example 50-200 $\mu$m thick and deposited directly on an entrance surface 12 of the fibre optics device. An optical sensitive sensor 14 is diretly coupled to an output surface 15 of the fibre optics device. The cutting angle a may be choosen such that a length measuring L of an X-ray image projected on the luminescent layer 10 is reduced to a measuring 1 of the optical image projected on the sensor by fibres 24 of the fibre optics device. The reduction factor may be about 3 and for a practical device L can be about 12 mm reduced to 4 mm for the sensor measuring l. In the other direction thus square to the drawing surface as well the X-ray image as the sensor measuring may be about 5 to 10 mm. In order to avoid any direct view of the sensor 14 by the X-ray beam 4 an additional screening 26 can be added which also can act as protection against unwanted light impinging on the sensor.

Figure 3 shows in a kind of exploded view a cascade of two fibre optics devices 13, respectively 23. An X-ray image projected on a luminescent screen 10 covering the surface 12 of fibre optics device 13 is projected on a second face 15 of the device reducing the measuring L into measuring l. An entrance face 25 of the second fibre optics device 23 is preferably directly coupled to the face 15 of device 13. For a good optical transmission at least one of the faces 15 or 25 is roughened such as described in US 4,247,165. The image projected on face 15 thus is accepted in fibre optics device 23 and transmitted to an exit face 26 thereof reducing a measuring B of the first optical image corresponding with the X-ray image into a measuring b to which a dimension of the optical sensor 14 is adapted or practically spoken the reduction is adapted to the relevant measuring of sensor 14 which is fixed to the output face 26 of fibre optics elements 23.

In all the examples described the fibre optics device is used to reduce the image dimensions. It may be obvious of course due to optical reversebility to use an optical device according to the invention to enlarge the image dimensions. Both actions can also be used without a luminescent layer thus acting only on light optical images. The detection system can be provided with an electrical or electronic sensor to detect the start of X-ray irradiation for automatically starting the read-out of the image matrix.

## Claims

1. A dental X-ray detection system for detecting one or some teeth at a time comprising an optical sensitive sensor, fibre optically coupled to a luminescent screen, characterized in that the detection system comprises a fibre optics device having a first surface obliguely cut with respect to the fibres, introducing an image area reduction of an image projected on that surface and a second surface of the fibre optics device optically coupled to the first surface.

2. A dental X-ray detection system as claimed in Claim 1, characterized in that the fibre optics device reduces a long axis of a rectangular X-ray image adapted to a short axis of a rectangular optical sensor.

3. A dental X-ray detection system as claimed in Claim 1 or claim 2, characterized in that it comprises a cascade of two fibre optics devices each reducing an axis of an X-ray image independently.

4. A dental X-ray detection system as claimed in Claim 1, 2 or 3, characterized in that one or each of the fibre optics devices reduces an axis of an X-ray image with about a factor three.

5. A dental X-ray detection system as claimed in any one of the preceding Claims, characterized in that the optical sensitive sensor is shaped as a CCD matrix sensor.

6. A dental X-ray detection system as claimed in any one of the preceeding Claims, characterized in that the luminescent layer is directly deposited on relevant end faces of the fibre optics devices.

7. A dental X-ray detection system as claimed in any one of the preceding Claims, characterized in that a control unit thereof is provided with a sensor which automatically activates the detection system on X-ray capture.

8. A dental X-ray detection system as claimed in any one of the preceding Claims, characterized in that gladding glass and/or extra mural absorbing elements of fibre optics device contains X-ray absorbing material.

9. A dental X-ray detection system as claimed in any one of the proceeding claims characterized in that the read out second surface of the fibre optics device is obliguely cut with respect to the fibres.

10. A dental X-ray apparatus comprising a detection system as claimed in ay one of the preceeding Claims.

FIG.1

FIG.2

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 279 294 (SIEMENS AG) <br> * column 8, lines 16-53; figures 2,3,8,9 * | 1,2,5,6 | A 61 B 6/14 |
| A | | 3,4,7 | |
| | --- | | |
| X | EP-A-0 285 214 (N.V. PHILIPS GLOEILAMPENFABRIEKEN) <br> * column 3, line 12 - column 4, line 3; figure 2 * | 5,6 | |
| A | | 1,2,7 | |
| | --- | | |
| A,D | FR-A-2 578 737 (F. MOUYEN et al.) <br> * abstract; page 2, lines 16-24; figure * | 1,8 | |
| | ----- | | |

TECHNICAL FIELDS
SEARCHED (Int. Cl.5)

A 61 B 6/00
G 02 B 6/00
G 01 T 1/00

The present search report has been drawn up for all claims

| Place of search | Date of completion of the search | Examiner |
|---|---|---|
| BERLIN | 01-03-1990 | WEIHS J.A. |

EPO FORM 1503 03.82 (P0401)